# EUROPEAN PATENT SPECIFICATION

(11) **EP 1 854 501 B1**
(45) Date of publication and mention of the grant of the patent: **08.12.2010**
(21) Application number: 06076323.2
(22) Date of filing: 29.06.2006
(51) Int. Cl.: A61M 25/06, A61M 25/00, A61N 5/00

(54) **Anchoring catheter needle for internally irradiating a tumor in a body part**
Verankerungskatheternadel für innere Bestrahlung eines Tumors in einem Körperteil
Cathéter à aiguille ancrable pour irradiation d'une tumeur dans une partie du corps

(30) Priority: 10.05.2006 NL 1031786
(43) Date of publication of application: 14.11.2007
(73) Proprietor: Isodose Control Intellectual Property B.V. i.o., 3905 PE Veenendaal (NL)
(72) Inventor: van't Hooft, Eric, 2930 Brasschaat (BE)
(74) Representative: van Loon, C.J.J.

(56) References cited:
- WO-A-93/25155
- WO-A-2004/030740
- US-A- 3 952 742
- US-A- 4 790 817
- US-A- 5 569 213
- US-A1- 2004 133 254

## Description

The invention relates to a catheter needle for internally radiating a tumor in a body part.

In some therapeutic treatments, in particular irradiating patients for the treatment of tumors, it is necessary to provide a plurality of catheters in the tissue. In the treatment of prostate tumors in men, for instance, it is common practice to pre-puncture the patient's skin with a sharp metal point before the plastic needles are provided in the prostate. In these needles, at a later time, radioactive sources are positioned in the catheters for irradiating a tumor located around them without overloading the surrounding tissue. For the patient, a treatment with such a plastic needle is extra traumatic, due to the blunt point being pressed through the usually harder tissue with force, which is very painful so that complete anesthesia is often required. Further, pressing a blunt needle with great force can result in wound fluid formation.

It is an object of the invention to be able to provide an alternative to above-mentioned treatment method and, to this end, the invention provides an apparatus according to claim 1. In particular, the invention provides a catheter needle which is shaped as an elongated tube of a first material with closed end and a sharp point from a second material which is harder than the first material.

WO2004/030740 discloses a flexible polymer needle catheter. It comprises an inflatable tube that is able to adaptively angle the needle tip portion to a wide range of positions to target a vessel wall. The needle catheter is used to deliver a therapeutic agent or drug.

WO9325155 discloses an optical fiber having a diffuser type silicone tip provided with a metal tip jacket. The tube is a solid flexible tip illuminator having a complex illumination ensemble.

US20040133254 discloses an inflatable balloon catheter. The catheter has a solid design and is intended for RF radiation purposes.

US4790817 shows a hollow tube with an open end and a retractable grooved stylet. The tube is provided of an expandable hydrophilic polymer.

US3952742 concerns a hollow open ended catheter having a retractable needle.

US5569213 shows a polymeric flexible piercing member with a piercing tip. Essentially, these parts slide in relation to the piercing member.

GB2150837 shows an anchoring catheter structure for anchoring in a bodily cavity such as a bladder having an open tube end suitable for retrieving fluid from the bladder.

FR2343488 in addition discloses an anchoring structure having an inner tube through which a pointed trocar can be guided through a welded end part.

The invention will now be explained in more detail with reference to the description of the Figures, in which:
Fig. 1 shows a conventional catheter;
Fig. 2 shows an anchoring needle;
Fig. 3 shows examples of the apparatus according to the invention;
Fig. 4 shows a side elevational view of the apparatus in use.

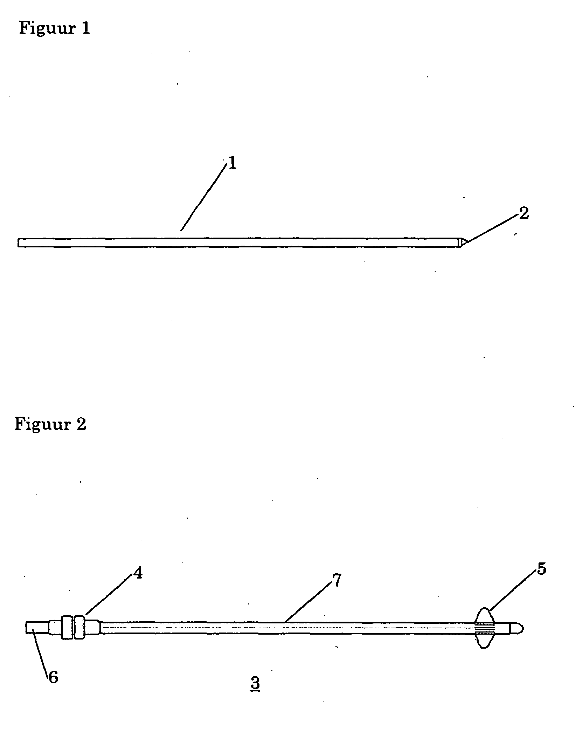

Fig. 1 shows a conventional catheter 1. The catheter 1 contains a conventional catheter needle 2 which is fed through the catheter 1, which needle 2 can be inserted into the tissue with catheter 1. After providing the needle 2 in the tissue, it is removed from the catheter 1 by, for instance, withdrawing it at the back side and the catheter 1 can be connected to a brachytherapy apparatus (not shown), which feeds a radioactive source through the catheter 1 to the position to be irradiated in the tissue. Conventional brachytherapy provides the placement of a number of catheters in a tissue to be irradiated in predetermined positions, such that the tissue receives a predetermined irradiation dose from radioactive sources which are provided in the catheters.

Fig. 2 shows a conventional anchoring needle 3 which can be used for fixation in a tissue. Such anchoring needles known per se typically have an anchoring mechanism for anchoring in the tissue. Typically, such needles may be provided with a double wall of which the outer wall has multiple parallel indentations all round so that, upon pulling the inner catheter, the outer wall bends outwards at the location of the indentations so that this catheter locks in the tissue. The anchoring needle 3 typically comprises a fixing part 4 and a laterally movable anchoring element 5 to be anchored in the body part, which element can be fixed by rotation or displacement of the fixing part. This may, for instance, be achieved by means of a central part 6, which can be moved in a tube part 7 and which presses the anchoring element 5, formed by a strip-shaped structure provided in the tube part, laterally outwards upon displacement. Fig. 2 shows the position pressed outwards of the anchoring element 5.

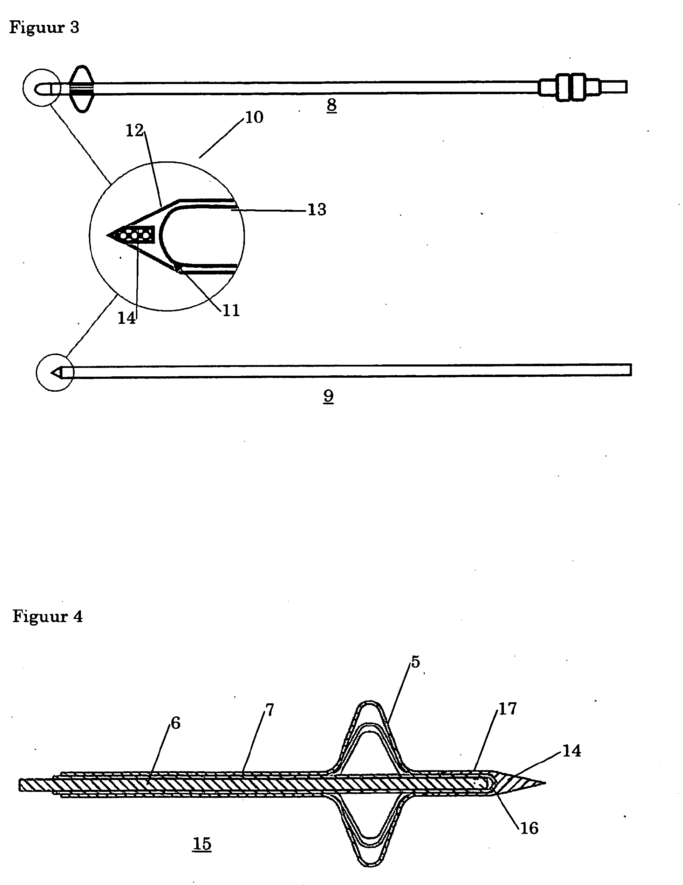

Fig. 3 shows examples of catheter needles 8 and 9. In particular, the detailed view 10 shows end 11 of catheter needle 8 with anchoring and of catheter needle 9 without anchoring. End 11 is formed by a closed end 12 of a hollow guide tube 13.The tube material is a plastic. In the closed end 12, a sharp point 14 is placed, in particular from a metal or ceramic material. As an example, the type of metal titanium can be chosen. Plastic points 14 have the property that they do not have the same sharpness as metal or ceramic points. This problem is solved by placing a sharp point 14 from a hard material in the plastic end 12 of the catheter tube 8 and 9, respectively. Thus, the catheter tube 8 and 9, respectively, has the same sharpness as a completely metal needle. Thus, the catheter tube 8 and 9, respectively, can puncture the skin directly, without pre-puncturing or other aids, and can be inserted into tissues difficult to penetrate for plastic needles, such as a prostate.

Fig. 4 shows the embodiment according to the invention. The locking needle 15 has an anchoring element 5 folding outwards. The Figure shows an inner tube 6 and an outer tube 7 movable around it, which are glued together to near the end to an end 16 around which a sleeve-shaped element 17 is cased as a connecting element, which tapers on the front side, i.e. the side facing away from the catheter needle, into a sharp point 14. The sleeve-shaped element is designed with a sharp needle and designed in titanium. Other suitable materials may of course be used as well. Another variant may be a separate (sleeve-shaped) casing element or connecting element, which forms a connection between a (separate) point and the end of the catheter needle.

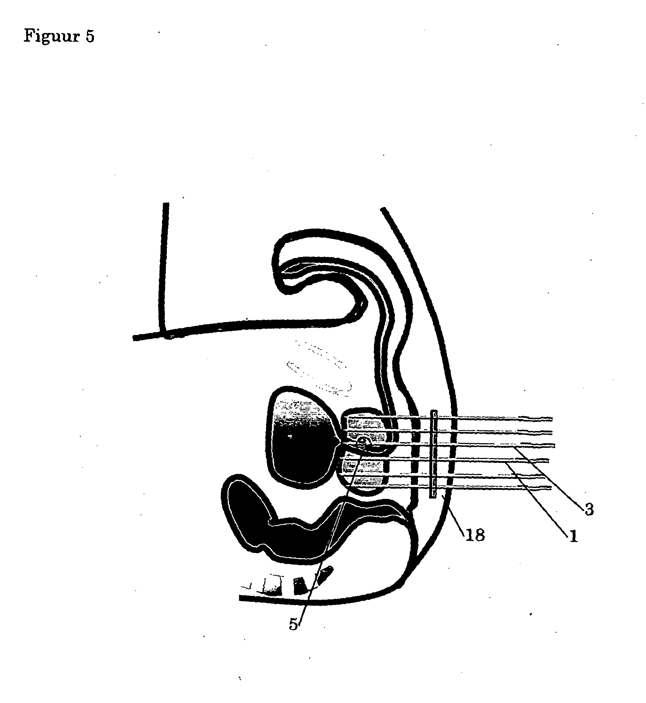

Fig. 5 shows a schematic view of a catheter needle in use. The catheter needle 1 may be anchored to a so-called template 18, which serves to position catheter needles 1. Catheter needles 3 involve the anchoring element 5 discussed with reference to Fig. 2. Both catheter needles 1 and 3 may be suitable for use as a guide tube for guiding a radioactive source (not shown).

The invention is not limited to the embodiment shown in the drawing, but may also comprise alternatives or variants thereof which fall within the scope of the following claims. Such variants are understood to fall within the claims defined as follows.

## Claims

1. An anchoring catheter needle (15) for internally irradiating a tumor in a body part, wherein the catheter needle comprises an elongated outer tube (7) from a first material, wherein the needle (15) further comprises an inner tube (6), the outer tube comprising a strip shaped anchoring structure (5) that is laterally movable relative to the inner tube (6), the inner tube (6) and outer tube (7) connected by a casing element (14) **characterized in that** the inner tube has a closed end (16) and **in that** the casing is formed as a sleeve-shaped element cased around the closed end (16) and having a sharp point (14) that is formed from a second material which is harder than the first material.

2. A catheter needle according to claim 1, **characterized in that** the sharp point (14) is from metal.

3. A catheter needle according to claim 1, **characterized in that** the sharp point (14) is from titanium.

4. A catheter needle according to claim 1, **characterized in that** the sharp point (14) is from a hard plastic.

5. A catheter needle according to claim 1, **characterized in that** the sharp point (14) is from ceramic material.

6. A catheter needle according to claim 1, **characterized in that** the sharp point (14) is cased in the first material.

## Patentansprüche

1. Verankerungskatheternadel (15) zur internen Bestrahlung eines Tumors in einem Körperteil, wobei die Katheternadel eine längliche äußere Röhre (7) aus einem ersten Material aufweist und wobei die Nadel (15) ferner eine innere Röhre (6) aufweist, wobei die äußere Röhre eine streifenförmige Verankerungskonstruktion (5) aufweist, die in Bezug auf die innere Röhre (6) seitlich bewegbar ist, wobei die innere Röhre (6) und die äußere Röhre (7) durch ein Gehäuseelement (14) verbunden sind, **dadurch gekennzeichnet, dass** die innere Röhre ein geschlossenes Ende (16) hat, und **dadurch**, dass das Gehäuse als ein hülsenförmiges Element ausgebildet ist, das umgebend um das geschlossene Ende (16) angeordnet ist und eine scharfe Spitze (14) aufweist, die aus einem zweiten Material ausgebildet ist, das härter als das erste Material ist.

2. Katheternadel nach Anspruch 1, **dadurch gekennzeichnet, dass** die scharfe Spitze (14) aus Metall ausgebildet ist.

3. Katheternadel nach Anspruch 1, **dadurch gekennzeichnet, dass** die scharfe Spitze (14) aus Titan ausgebildet ist.

4. Katheternadel nach Anspruch 1, **dadurch gekennzeichnet, dass** die scharfe Spitze (14) aus einem harten Kunststoff ausgebildet ist.

5. Katheternadel nach Anspruch 1, **dadurch gekennzeichnet, dass** die scharfe Spitze (14) aus keramischem Material ausgebildet ist.

6. Katheternadel nach Anspruch 1, **dadurch gekennzeichnet, dass** die scharfe Spitze (14) in dem ersten Material aufgenommen ist.

## Revendications

1. Aiguille de cathéter à ancrage (15) destinée à l'irradiation interne d'une tumeur dans une partie du corps, l'aiguille de cathéter comprenant un tube extérieur allongé (7) réalisé en un premier matériau, l'aiguille (15) comprenant en outre un tube intérieur (6), le tube extérieur comprenant une structure d'ancrage en forme de bande (5) qui est mobile latéralement par rapport au tube intérieur (6), le tube intérieur (6) et le tube extérieur (7) étant reliés par un élément gainant (14), **caractérisée en ce que** le tube intérieur présente une extrémité fermée (16) et **en ce que** l'élément gainant se présente sous la forme d'un élément de type manchon engagé autour de l'extrémité fermée (16) et doté d'une pointe acérée (14) réalisée en un second matériau plus dur que le premier matériau.

2. Aiguille de cathéter selon la revendication 1, **caractérisée en ce que** la pointe acérée (14) est en métal.

3. Aiguille de cathéter selon la revendication 1, **caractérisée en ce que** la pointe acérée (14) est en titane.

4. Aiguille de cathéter selon la revendication 1, **caractérisée en ce que** la pointe acérée (14) est en plastique dur.

5. Aiguille de cathéter selon la revendication 1, **caractérisée en ce que** la pointe acérée (14) est en matériau céramique.

6. Aiguille de cathéter selon la revendication 1, **caractérisée en ce que** la pointe acérée (14) est logée dans le premier matériau.
